# EUROPEAN PATENT SPECIFICATION

(11) **EP 1 305 343 B1**
(45) Date of publication and mention of the grant of the patent: **12.10.2005**
(21) Application number: 01962874.2
(22) Date of filing: 26.07.2001
(51) Int. Cl.: C07K 16/46, C07K 14/31

(54) **PEPTIDES PRESENTED BY CELLS**
VON ZELLEN PRÄSENTIERTE PEPTIDE
PEPTIDES PRESENTS PAR CELLULES

(30) Priority: 03.08.2000 GB 0018901
(43) Date of publication of application: 02.05.2003
(73) Proprietor: Merck Patent GmbH, 64271 Darmstadt (DE)
(72) Inventor: CARR, Francis, J., Balmedie, Aberdeen AB23 8XU (GB); CARTER, Graham, By Newmachar, Aberdeenshire AB21 7XB (GB); HELLENDOORN, Koen, Newmarket CB8 7AT (GB)
(86) International application number: PCT/EP2001/008625
(87) International publication number: WO 2002/012899

(56) References cited:
- WO-A-00/34317
- WO-A-98/52976
- DE JONG A.: "Contribution of mass spectrometry to contemporary immunology" MASS SPECTROMETRIC REVIEWS, vol. 17, no. 5, 1998, pages 311-335, XP008005296 cited in the application
- RAPOSO G ET AL: "BETA LYMPHOCYTES SECRETE ANTIGEN-PRESENTING VESICLES" JOURNAL OF EXPERIMENTAL MEDICINE, TOKYO, JP, vol. 183, 1996, pages 1161-1172, XP002060486 ISSN: 0022-1007

## Description

The present invention relates to methods to determine peptides presented on the surface of mammalian cells following addition to the cells of a protein. The present invention also relates to diagnostic tests based on the determination of such peptides or modified molecules resulting from determination of such peptides, such as pharmaceutical entities preferably having specific biological activity and reduced or enhanced immunogenicity when compared with the corresponding non-modified molecules. The methods according to this invention are preferably established with tools using mass spectroscopy (MS).

Following the uptake of proteins by mammalian cells (or the production of specific proteins within cells), proteins are subsequently degraded and, commonly, peptide fragments of such proteins appear at the cell surface, often associated with other proteins. In particular, peptide fragments of a protein can be degraded and certain peptides subsequently become associated with major histocompatability complex (MHC) molecules which, in many cases, can be recognised by T cells in order to initiate an immunological response. Such an immunological response can be beneficial, for example where the immune system counteracts cells producing the specific protein from where the peptide fragments are derived, or can be detrimental, for example where the immune system produces antibodies which bind to the specific protein and limit its activity (such as with a pharmaceutical protein).

To date, for a given protein, it has been impossible to predict exactly at which locations within the protein that degradation takes place such that the exact peptides, which appear on the cell surface, cannot be reliably predicted. In the case of MHC molecules, there has been some success in predicting the motifs of peptides which bind to MHC but, in practice, some of these peptides are degraded before reaching MHC molecules and prediction of which peptides are degraded is not reliable. Thus, the standard method for detection of peptides on the cell surface is elution of such peptides followed by their sequence determination. Such methods are not routine and are also impractical where analysis of cell surface peptides on multiple cell types or on multiple MHC molecules is required.

It is an object of the present invention to provide for methods for detecting peptides on the surface of cells especially presented by multiple MHC molecules. Furthermore, it is an object of the invention to use such information either for design of a pharmaceutical molecule, or for design of a vaccine molecule, or for a diagnostic test.

For a pharmaceutical molecule, it is a particular object of the present invention to identify peptides presented by MHC molecules following uptake or production of a protein by cells and, using this information, to alter the protein such that such peptides are no longer presented.

For a vaccine molecule, it is a particular object to identify peptides presented by MHC molecules following uptake or production of a protein by cells and to use one or more of such peptides within a vaccine molecule in order to stimulate the immune system.

For both pharmaceutical and vaccine molecules, it is a particular object of the present invention to identify peptides presented by many different MHC molecules to encompass different allotypic and genotypic variants throughout different populations.

For a diagnostic test, it is a particular object to determine peptides presented by MHC molecules following uptake or production of a protein by cells and to use such determination as the basis for a test for the detection of a biological or physiological event, for example for the detection of an infection.

For a diagnostic test, it is a particular object of the present invention to identify peptides presented by cells of a test individual.

The present invention provides for the accurate and comprehensive analysis of peptides on the surface of cells and in particular peptides in association MHC molecules. Determination of the profile or identity of peptides on the cell surface will be especially performed mass spectrometry (MS).

Others have previously purified MHC/peptide complexes for particular purposes. Indeed for both MHC class I and class II molecules, methods for their immunological enrichment and purification have been instrumental in enabling the elucidation of the peptide-MHC binding interaction and enabled the elucidation of MHC binding motifs. Pertinent examples include US5,989,565 and US,6,077,519 wherein are provided methods for the identification of autologous T-cell epitopes by acid elution of peptides from the surface of patient tumour cells or dendritic cell populations. The eluted peptide sequences have utility in the design of synthetic peptides for the production of vaccines.

Similarly, Salter et al [US5,487,982] provide genetically engineered temperature-sensitive MHC class I molecules enabling the facile removal of peptides bound to the mutant MHC class I molecule from engineered cells grown *in vitro.*

Further examples of the art include Langlade-Demoyen et al [WO9744667] who exploit purified MHC-peptide complexes as affinity reagents for the enrichment of tumour specific T-cells for tumour immunotherapies *in vitro* and *in vivo* and other applications. Similarly, US5,763,585, US5,734,023 and others provide methods for the purification of particular MHC peptide complexes for use as therapeutic entities for example in the treatment of auto-immune diseases and, Deshpande et al [WO9740852] disclose modified peptides with enhanced binding to an MHC class II protein and where the whole complex is provided as a recombinant fusion protein also intended for use in the treatment of diseases associated with auto-reactive T cells.

WO9734143 and US5,792,604 provide methods for identifying MHC class I restricted antigens endogenously processed by cellular secretory pathway. This biological assay requires primed cytotoxic T cells and a source of "indicator" target cells onto which lysis is directed by the presence of processed peptides secreted into the medium. The scheme has utility in the identification of substances able to influence the endogenous processing pathways of the donor cells. Other complex biologically based schemes for the identification MHC class I and MHC class II restricted T-cell epitopes include WO00/67761 where is provided a method exploiting genetic vaccination and isolation of dendritic cells and splenocytes for conducting biological T-cell activation assay *in vitro.*

The present inventors have recognised that MS-based instruments may be applied to the analysis of whole cells or cell extracts and the data incorporated into a pathway for the rational development of therapeutic molecules or diagnostic assays.

Large and complex molecules of biological origin are amenable to analysis using mass-spectroscopy (MS), but only following their ionisation. Two alternative approaches to the ionisation of biological molecules for MS have become the recognised technologies in this area. These are electrospray ionisation (ESI) and matrix assisted laser de-sorption ionisation (MALDI). In ESI the sample is pumped through a charged narrow capillary and nebulised by a parallel gas flow until ultimately electrostatic repulsion causes desorption of the analyte ions into the mass spectrometer. ESI is a continuous flow technique and as such is readily connected to up-stream sample separation technologies such as liquid chromatography (LC) or capillary electrophoresis (CE) [Cao & Moini (1998) *Am. Soc. Mass Specrom*. 9: 1081-1088]. These features are in contrast to MALDI-MS techniques where the sample is embedded within a crystalline matrix material deposited on the sample plate of the instrument. lonisation is achieved by laser excitation of the matrix and the desorbed analyte ions are accelerated into the mass analyser. MALDI is therefore a discontinuous process and multiple laser pulses are used to produce ions with data cumulatively collected in the mass analyser.

For MALDI, mass analysis is most usually conducted by a time of flight (TOF) instrument where the flight time from the ionising laser pulse to detection is measured enabling calculation of the mass to charge ratio (*m*/*z*). Such detectors are subject to multiple technical refinements in different commercial instruments. Some models enable analysis of post-source decay ion fragments for peptide sequence derivation and other structural determinations. The continuous flow nature of ESI based instruments result in the use of scanning analysers (e.g. quadrupole or magnetic sector mass analysers) although other instrument formats may be exploited. A typical format is the tandem MS system whereby dual mass analysers are arranged in tandem and interconnected via a "collision cell" containing molecules of inert gas. The latter feature enables generation of collision induced decay ions and the determination of peptide sequence from the given input sample. Multiple and hybrid instrumental formats may be arranged and exploited for the analysis of cell surface (MHC) associated peptides.

Others have used MS in the analysis of peptides eluted from MHC and in particular MHC class I molecules as reviewed by Cox et al [Cox, A. L. et al (1997) pp 141-160 in *MHC1:A Practical Approach* Eds Femadez N. & Butcher G. Oxford University Press, Oxford, UK] and a more recent review of this area is provided by De Jong [De Jong, A. (1998) *Mass Spectrometry Reviews* 17: 311-335]. Thus Ovysyannikova et al [Ovysyannikova et al (2001) *J. Immunol. Methods* 246: 1-12], exploit MALDI-TOF in the analysis of self peptides acid eluted from MHC class II allele DRB1*0401 following treatment of the cells with measles virus vaccine. Similarly, Sickman et al [Sickman, A. et al (2000) *Analyst* 125: 569-573] describe the identification of MHC class II bound peptides from rat Langerhans cells using a combination of LC and MALDI-MS techniques.

The present invention however, incorporates and extends all such prior art by for the first time providing a generalised scheme for the elucidation of peptide species binding to MHC class I and MHC class II molecules using MS based instrumentation and in the first embodiment provides methods for the removal of the binding interaction by amino acid substitution within the peptide sequence for the purpose of developing a therapeutic protein with a reduced or absent ability to provide an MHC-mediated immune response on administration to a subject, most preferably a human subject. It is an objective of the present invention to provide a method for the development of a therapeutic molecule with reduced capacity to elicit an immune response upon administration to a subject.

It is an objective of the present invention to provide for methods for detecting peptides on the surface of cells and in particular where the peptides are in association with MHC molecules.

It is an objective of the present invention to provide for methods for detecting peptides formally in association with MHC molecules presented on the surface of cells.

It is an objective of the present invention to provide for methods for detecting peptides in association with multiple different MHC molecules present on the surface of cells.

It is an objective of the present invention to provide for methods for detecting peptides in association with MHC molecules whereby those peptides originate from an exogenous protein.

It is an objective of the present invention to provide for methods for detecting peptides in association with MHC molecules whereby those peptides originate from an endogenous protein.

It is an object of the present invention to provide for methods for detecting peptides on the surface of a cell following a process whereby the cells of interest have been contacted with an exogenous protein or an exogenous microrganism such that their repertoire of surface peptides is different from the repertoire displayed on otherwise identical reference cells but which have not been contacted with the exogenous protein.

The exogenous protein may be a purified preparation extracted from a mammalian source such as a cell line or tissue *ex vivo,* or the protein may be a recombinant preparation purified from any biological source engineered to express said protein. The protein may be representative of a naturally occurring protein or a fusion of one or more naturally occurring proteins. The protein may be *in vitro* derived being an assemblage of naturally occurring elements or wholly synthetic or designed and have no counterpart in nature.

The protein may be exemplary of a class of proteins such as antibody molecules and their derivatives, cytokines, growth factors, leukotrines, haemostatic factors or may be a cell toxin or have enzymatic capacity or function. Most preferably the protein is a therapeutic protein.

It is recognised that the efficacy of several therapeutic proteins has been limited by the induction of unwanted immune reactions to the therapeutic protein. Examples include monoclonal antibodies [Schroff, R. W. et al (1985) *Cancer Res.* 45: 879-885; Shawler, D.L. et al (1985) *J. Immunol*. 135: 1530-1535] and also some proteins of human origin such as granulocyte-macrophage colony stimulating factor [Wadhwa, M. et al (1999) *Clin. Cancer Res*. 5: 1353-1361] and interferon alpha 2 [Russo, D. et al (1996) *Bri. J. Haem.* 94: 300-305; Stein, R. et al (1988) *New Engl. J. Med.* 318: 1409-1413] amongst others. There is therefore a significant need for methods able to identify determinants involved in the induction of an immune response to a therapeutic protein.

A principal factor in the induction of an immune response is the presence within the protein of peptides that can stimulate the activity of T cell via presentation on MHC class II molecules, so-called T cell epitopes. In order to eliminate or reduce immunogenicity, it is desirable to identify and remove T cell epitopes from the protein. It is an object of the present invention to provide for methods to enable the detection and identification of T cell epitopes.

Exogenous proteins are engulfed and processed for presentation in association with MHC class II molecules of the DR, DQ or DP type. MHC Class II molecules are expressed by professional antigen presenting cells (APCs), such as macrophages and dendritic cells amongst others. The ability of a peptide to bind a given MHC class II molecule for presentation on the surface of an APC is dependent on a number of factors most notably its primary sequence. This will influence both its propensity for proteolytic cleavage and also its affinity for binding within the peptide binding cleft of the MHC class II molecule. The MHC class II / peptide complex on the APC surface presents a binding face to a particular T cell receptor (TCR) able to recognise determinants provided both by exposed residues of the peptide and the MHC class II molecule. In the art there are procedures for identifying synthetic peptides able to bind MHC class II molecules. Such peptides may not function as T cell epitopes in all situations particularly *in vivo* due to the processing pathways or other phenomena. Also in the art there are computational methods for predicting potential T-cell epitopes or recognising sequence motifs in experimentally determined T-cell epitopes. Schemes include techniques to predict MHC class II-binding peptides as provided in WO98/52976 where a computational threading approach identifies peptide sequences with the potential to bind only a sub-set of possible human MHC class II DR allotypes.

T cell epitope identification is the first step to epitope elimination as recognised in WO98/52976 and WO00/34317. In these teachings, predicted T cell epitopes are removed by the use of judicious amino acid substitution within the primary sequence of the therapeutic protein. It is an objective of the present invention to provide methods for the development of modified proteins in which the immune characteristic is modified by means of reduced numbers of potential T-cell epitopes. The identified sequences being present on the surface of MHC bearing cells or bound to MHC preparations following the natural intracellular processing events present within any competent APC or similar cell. Moreover, peptides identified under the scheme of the present invention can be detected from any MHC allotype or a mixture of allotypes including for MHC class II, those of the DR, DQ or DP specificities.

Whilst the present invention enables the detection of peptides displayed in the surface of cells and in particular in association with MHC molecules of the class I and class II designations, it is not intended to be limited to whole cells but also includes the analysis of peptides on the surface of exosomes. Peptide-MHC complexes are present in high concentration on the surface of exosomal vesicles derived from cells normally expressing MHC class II molecules. Under certain circumstances the output of exosomes may be increased from the surface of an APC and there are recognised procedures for the enrichment or isolation of exosomes [Raposo, G. et al (1996) *J. Exp. Med.* 183: 1161-1172; Clayton, A. et al (2001) *J. Immunol. Methods* 247: 163-174]. Analysis of APC preparations and preparations of exosomal particles derived from APC equally therefore fall under the scope of the present invention.

In summary the invention relates to the following issues:
- A method for the development of a pharmaceutical protein or polypeptide or vaccine antigen having a specific biological activity and a reduced or enhanced immunogenicity than any non-modified protein or polypeptide having the same biological activity, by
   (i) contacting cells with said protein, or polypeptide, generating a repertoire of surface peptides on the cells or exosomal vehicles thereof, which is different from the repertoire of surface peptides displayed on reference cells which have not been contacted,
   (ii) analyzing said cells or exosomal vesicles thereof, for peptides bound on the surface of said cells or exosomal vesicles thereof and,
   (iii) assigning said peptides to the sequence of the pharmaceutical protein or polypeptide or vaccine antigen according to standard methods.
- A corresponding method further comprising the following steps:
   (iv) modifying said peptides, in order to alter their binding to MHC molecules, and
   (v) constructing sequence variants of the final pharmaceutical protein or polypeptide by incorporating one or more of the modified peptide sequences within the sequence of the protein or polypeptide molecule according to standard methods.
- A corresponding method, wherein the analysis of said cells or exosomal vehicles thereof of step (ii) is performed by using mass spectroscopy (MS), preferably MALDI-MS and ESI-MS.
- A corresponding method, wherein the pharmaceutical protein or polypeptide is modified such that one or more of said peptides are no longer bound after contacting cell with said protein or polypeptide.
- A corresponding method, wherein the peptides bound on the surface of the cells or exosomal vesicles according to step (i) are in association with MHC molecules and wherein the analysis of said cells or exosomal vesicles thereof according to step (ii) is performed by using MS.
- A corresponding method, wherein the peptides bound on the surface of the cells or exosomal vesicles according to step (i) are products of an intracellular peptidase and trafficking pathway.
- A corresponding method for the development of a pharmaceutical protein or polypeptide having a reduced immunogenicity, wherein the modification of the immunogenic peptides is performed by eliminating or reducing their binding to MHC molecules, optionally by testing the modified peptides for binding to the cell surface as indicated in claim 1.
- A corresponding method, wherein the elimination or reduction of the binding of the peptides to MHC molecules is performed by substituting, inserting or deleting one or more amino acid residues within the sequence region of the peptide within the pharmaceutical protein or polypeptide.
- A method for the development of a pharmaceutical protein or polypeptide having an enhanced immunogenicity, wherein the modification of the peptides are performed by enhancing their binding to MHC molecules, optionally by testing the modified peptides for binding to the cell surface as indicated in claim 1.
- A corresponding method, wherein the enhancement of the binding of the peptides to MHC molecules is performed by substituting, inserting or deleting one ore more amino acid residues within the sequence region of the peptide increasing the activity of the peptide to act as a T-cell epitope, and / or broadening the range of MHC types for which the T-cell epitope is restricted, and / or combining several otherwise disparate epitopes into a single entity.
- A method for the development of a vaccine by
   (i) contacting cells with a protein or polypeptide or micro-organism having immunogenic activity, generating a repertoire of surface peptides on the said cells or exosomal vesicles thereof, which is different from the repertoire of surface peptides displayed on reference cells which have not been contacted,
   (ii) analyzing said cells or exosomal vesicles thereof, on the surface of which peptides are bound,
   (iii) assigning said peptides to the sequence of the protein or polypeptide according to standard methods, and
   (iv) constructing sequence variants of the final pharmaceutical vaccine by incorporating one or more of the peptides within the sequence of the vaccine molecule according to standard methods, wherein the analysis of said cells or exosomal vesicles therein is performed by using MS.
- A corresponding method using human cell lines engineered to produce MHC molecules.
- A corresponding method in which the parent (non-engineered) cell line produces no MHC molecules.
- A corresponding method in which the parent cell line produces no MHC class I molecules.
- A corresponding method in which the parent cell line produces no MHC class II molecules.
- A corresponding method, wherein non-human cells which do not produce their own MHC molecules are engineered to produce MHC molecules and are used as indicated.
- A corresponding method, wherein the MHC molecules derive from MHC class II.
- A corresponding method, wherein the MHC molecules are HLA-DR, HLA-DQ and HLA-DP.
- A corresponding method, wherein the MHC molecules derive from MHC class I.
- A corresponding method, wherein a combination of cell lines or cell samples providing a comprehensive mixture of different MHC allotypes and genotypes is used.
- A corresponding method, wherein the peptides originate from an exogenous protein or micro-organism.
- A corresponding method, wherein the peptides originate from an endogenous protein.
- A corresponding method, wherein human dendritic cells, or exosomal vesicles thereof, which after addition of the protein or polypeptide or micro-organism present peptides on MHC molecules, are used.
- A corresponding method, wherein human antigen presenting cells, or exosomal vesicles thereof, which after addition of the protein or polypeptide present peptides on MHC molecules, are used.
- A corresponding method, wherein the MHC molecules have been enriched prior to peptide analysis.
- A corresponding method, wherein the peptides are eluted from the cell surface prior to analysis.
- A corresponding method, wherein the peptides are eluted from MHC molecules prior to analysis.
- A corresponding method, wherein MALDI-MS is used.
- A corresponding method, wherein ESI-MS is used.
- A method for the development of a diagnostic test by
   (i) analyzing appropriate human cells for surface peptides, and
   (ii) either, (a) producing a profile of peptides which appear on the cell surface and, optionally, comparing with other profiles to identify an abnormality or disease associated with the human cells, or, (b) determining the sequence of specific peptides on the cell surface as a means to determining specific peptides which might used to identify an abnormality or disease associated with the human cells.
- Use of a protein or polypeptide obtained by any of the methods described above as a pharmaceutical therapeutic entity.
- Use of a protein or polypeptide obtained by any of the methods described above as a vaccine.
- Use of a protein or polypeptide obtained by any of the methods described above as a pharmaceutical therapeutic entity having reduced immonogenicity.
- A method for detecting peptides on the surface of cells or exosomal vesicles thereof which derive from a protein or polypeptide by
   (i) contacting cells with said protein, or polypeptide or a gene coding for this protein or polypeptide, generating a repertoire of surface peptides on that cells or exosomal vesicles thereof, which is different from the repertoire of surface peptides displayed on reference cells which have not been contacted,
   (ii) analyzing said cells or exosomal vesicles therein, on the surface of which said peptides are bound and
   (iii) assigning said peptides to the sequence of the protein or polypeptide according to standard methods, wherein the analysis if the cells or exosomal vesicles is performed by MS, preferably MALDI-MS.

The process according the scheme under the first major embodiment of the present invention is therefore to apply the methods herein to:
1. Identify one or more processed T cell epitopes within the amino acid sequence of a target protein.
2. Design new sequence variants of the target protein with one or more amino acids within the identified potential T cell epitopes modified in such a way to substantially reduce or eliminate the activity of the T cell epitope as determined by the binding of the peptides to MHC molecules or whole cells or other means. Importantly, such sequence variants are created in such a way to avoid creation of new potential T cell epitopes by the sequence variations unless such new potential T cell epitopes are, in turn, modified in such a way to substantially reduce or eliminate the activity of the T cell epitope.
3. Construct such sequence variants and testing said variants in order to identify one or more variants with desirable properties.

According to this scheme, a number of variant proteins will be produced and tested, most preferably by recombinant DNA techniques although other procedures including chemical synthesis may be contemplated. It is anticipated that single amino acid substitutions within a given potential T cell epitope will be made to eliminate the epitope. Combinations of substitution within a single epitope may be contemplated

In a major aspect of the present invention, a population of mammalian cells is incubated with a test protein or transfected with a gene encoding a test protein and, following uptake or expression of the protein and its degradation, the profile or identity of peptides on the cell surface is determined. In particular, MHC molecules, especially multiple MHC molecules, will present such peptides. As one aspect of this embodiment, it is important to analyse peptides presented on many different MHC molecules and therefore the analysis is preferably undertaken on multiple cell populations encompassing a very high proportion of the MHC allotypes and genotypes that are encountered in the world population. Such cell populations can either be obtained by sampling multiple cell populations from the world population or by using cells that have been engineered to produce multiple MHC types. Of particular use are cell lines where no endogenous MHC molecules are produced and which display a low background of peptides on the cell surface.

Due to the large number of human MHC allotypes and genotypes each with the capacity to bind to a different profile of peptides from any given protein, previous approaches to the comprehensive identification of T cell epitopes as described above, have comprised primarily predictive approaches, rather than either biochemical (where peptides are tested for binding to large numbers of MHC types) or biological approaches (primarily where peptides are tested for activation or stimulation of T cells). For peptides that bind to MHC class II, such predictive approaches include motif and artificial neural network techniques whereby large numbers of sequences of T cell epitopes are analysed to determine specific combinations of amino acids common to these epitopes for subsequent predictive analysis of other peptides. However, for MHC class II, such predictive approaches are limited and will often miss a proportion of T cell epitopes (false negatives) or will assign certain peptides to be T cell epitopes when, in practice, they lack such an activity (false positives). A further major limitation of predictive approaches and, also, biochemical and biological approaches with synthetic peptides, is the inability to account for processing of the given protein that can influence which peptides are presented by MHC molecules. Thus, improved methods are needed for the accurate and comprehensive identification of T cells epitopes without appreciable false negatives or false positives and, as such, the present invention provides for such improvement.

For the development of a human pharmaceutical protein according to the first embodiment of the present invention, a particular application is to generate a protein whereby epitopes for activation and/or stimulation of helper T cells are removed. In this case, the primary interest is peptides presented by MHC class II. For development of a pharmaceutical protein that is effective in a high proportion of humans, it is necessary to identify peptides within the protein which can be presented by one or more of the numerous MHC allotypes and to then alter these peptides to remove the capacity for MHC binding. Previously, the comprehensive identification of peptides presented by MHC class II has been limited by the difficulty in accurately predicting such peptides and in predicting peptides which bind to one or more of the large number of different allotypic and genotypic human MHC class II molecules. In comparison to peptides which bind to MHC class I where prediction is more reliable, peptides which bind to MHC class II are more variable in length and have amino acids outside the main binding pockets which have a more significant effect on peptide binding. Thus, for MHC class II, there is a need for more accurate and comprehensive methods than currently available for predicting or detecting peptides which bind to one or more of the numerous MHC class II allotypes and genotypes in order to comprehensively identify potential T cell epitopes. Major benefits of the availability of such an approach is in the generation of pharmaceuticals with all major (or all) T cell epitopes identified and then removed.

A preferred method for the development of a pharmaceutical using the present invention comprises the following steps:
1. for the test protein for potential use as a pharmaceutical, add the protein to a selection of human cell lines or human cell samples
2. after an appropriate period of time, analyse the cells for surface peptides particularly using MALDI-MS directly on cells or, alternatively, using MALDI-MS on cell fractions especially MHC fractions, or alternatively, eluting peptides from the cell surface prior to analysis particularly by MALDI-MS or ESI-MS.
3. from the given protein, assign peptides which appear on the cell surface to the test protein
4. modify peptides which appear on the cell surface in (2) to eliminate binding to MHC molecules, in some cases by testing modified peptides for binding to the cell surface (as above)
5. generate the final pharmaceutical protein molecule by incorporating appropriate modifications to one or more peptides within the protein sequence to eliminate binding to MHC molecules

According to the second major embodiment of the present invention, there is provided a scheme whereby a vaccine molecule or molecule able to function as a vaccine may be developed. The vaccine is most preferable for use in humans although the scheme may equally be applied to the development of vaccine molecules for the treatment or prevention of disease in non-human species. For the development of a vaccine using the present invention, a particular application is to generate a peptide or protein whereby major epitopes for activation and/or stimulation of T cells are present, primarily epitopes for helper T cells but also, for applications requiring cell killing, epitopes for cytotoxic T cells (primarily MHC class I restricted). For development of a vaccine that is effective in a high proportion of humans, it is necessary to identify peptides within the protein that can be presented by one or more of the numerous MHC allotypes and genotypes and to then select from these peptides one or more epitopes for inclusion in the final vaccine molecule. Such epitopes can comprise peptides restricted by MHC class I or MHC class II or both. The present invention provides for such identification of peptides presented by MHC class I and class II and is thus the basis for development of more effective vaccines.

A major distinction between MHC class I and MHC class II epitopes is the principal origin of the protein from which the peptide in the MHC-peptide complex is usually derived. It is recognised that exogenous proteins give rise predominantly to peptides in association with MHC class II, whereas endogenous proteins expressed from within a cell are processed according to a different peptidase and intracellular trafficking pathway to result predominantly in association with MHC class I molecules on the cell surface. For either exogenous or endogenous proteins, during the sequence of processing steps, certain peptide sequences from the protein that might normally bind to MHC may be destroyed so that certain T cell epitopes are not produced *in vivo*. For such peptides, predictive (or biochemical / biological) methods cannot take account of processing in the identification of T cell epitopes. It is a feature of the present invention that peptides identified from the cell surface are those that have been selected by the APC (or other cell) as capable of passage via the intracellular processing and trafficking pathways. As the invention is focussed to the identification of the real products of the processing events, the invention therefore reduces the rate of false positive and false negative epitopes identified using predictive or *in vitro* methods of epitope identification.

The process according the scheme under the second main embodiment of the present invention is therefore to apply the methods herein to:
1. Identify one or more T cell epitopes within the amino acid sequence of a target protein.
2. Design new sequence variants of the target protein with one or more amino acids modified in such a way as to either;
   i) increase the activity of the T cell epitope and /or
   ii) broaden the range of MHC types for which the T cell epitope is restricted as determined by the binding of the peptides to MHC molecules or whole cells or other means; and/or
   iii) combine several otherwise disparate epitopes into a single (smaller) entity.
3. Construct such sequence variants and testing said variants in order to identify one or more variants with desirable properties.

According to this scheme, a number of variant proteins will be produced and tested, most preferably by recombinant DNA techniques although other procedures including chemical synthesis may be contemplated.

A preferred method for the development of a vaccine using the present invention therefore comprises the following steps:
1. for the protein for potential use as a vaccine, add the protein to a selection of human cell lines or human cell samples
2. after an appropriate period of time, analyse the cells for surface peptides particularly using MALDI-MS directly on cells or, alternatively, using MALDI-MS on cell fractions especially MHC fractions, or alternatively, eluting peptides from the cell surface prior to analysis particularly by MALDI-MS or ESI-MS.
3. from the given protein, assign peptides which appear on the cell surface to the test protein
4. select one or more peptides which appear on the cell surface in (2) for use in the final vaccine molecule

It will be understood that, for the analysis of cell surface peptides for pharmaceutical or vaccine use, a combination of cell lines or human cell samples providing a comprehensive mixture of different MHC allotypes and genotypes could be employed and that such natural cell samples will provide a comprehensive mixture of MHC class I and/or class II restricted epitopes. Of particular use would be human dendritic cells (or exosomes) which can be activated after addition of the test protein to efficiently present peptides on MHC molecules. Alternatively, human cell lines can be engineered to expand their MHC repertoires primarily by transfection of genes encoding one more MHC types into these cells thus producing cells with multiple MHC types for analysis of cell surface peptides. Of particular use would be non-human cells which do not produce their own MHC molecules, such as mouse cells in which the murine MHC genes have been deleted or otherwise disabled. The analysis of cell surface peptides will also include the optional step of enriching MHC molecules, for example using immunoaffinity columns, prior to peptide analysis. For MHC class II, the method of the invention for analysis of cell surface peptides has the particular advantage of providing for analysis of binding to allotypes other than DR. Analysis of peptide presentation by such allotypes as DQ and DP has been very difficult as there are no predictive method available especially for DQ where both chains of the MHC molecule are thought to contribute to binding (in contrast to just the β chain in DR).

According to the third major aspect to the present invention, there is provided a scheme whereby the invention is applied to the development of a diagnostic test. A particular application of this embodiment is to generate a profile of peptides presented on the cell surface in order to identify the abnormal presence, absence or pattern of peptides that might indicate a particular disease or cellular insult.

It is therefore an object of the present invention to provide for methods for detecting peptides on the surface of a cell following a process whereby the cells of interest have been contacted with an exogenous protein or organism or other agent such that their repertoire of surface peptides is different from the repertoire displayed on otherwise identical cells but which have not been contacted with the exogenous protein or organism. In the practice of the invention, the presence of a "diagnostic peptide" or "indicator peptide" may be made in isolation by the registration of the indicator peptide mass in the mass spectrum of an MS instrument, or by record of the indicator peptide sequence in a CID (or similar) spectrum. Equally the presence of the indicator peptide may be indicated with reference to a mass spectrum derived from a reference sample not contacted with the exogenous protein or agent. Such a comparative analysis is of particular value in instances where the mass(es) of the indicator peptides are not known or predicted by any means. Comparative analysis may be conducted *in silico* by subtraction of identical mass peaks and would be or particular value where the loss of a particular mass peak is the diagnostic indicator. Others too have exploited MS techniques in comparative analysis of biological samples. However, in the case of Liotta et al [WO0049410], such comparative analysis is focussed to the protein content of individual cells obtained by laser capture micro dissection. This is distinct from the current invention, where the diagnosis (comparative, subtractive or otherwise) is according to the peptides detected from the surface and particularly peptides in association with MHC molecules on the cell surface.

Other diagnostic techniques using MS technology include Little et al [US,6,207,370] who disclose an MS based diagnostic procedure directed towards the identification of genetic disease, that is a constitutional feature of the individual not an acquired condition or state. The procedure can identify the mass of a target protein that is variable in the population dependent on the genetic constitution of the individual. A further diagnostic scheme is disclosed by Geng et al [Geng, M. (2000) *J. Chromatography A.* 870: 295-313] who have analysed MALDI-TOF spectra obtained from tryptic digests of serum protein samples. Their process detects "signature peptides" diagnostic of the presence of an analyte protein present in a complex mixture. This too is distinct from the current invention, where the diagnosis (comparative, subtractive or otherwise) is according to the peptides detected from the surface and particularly peptides in association with MHC molecules on the cell surface.

Therefore, according to the scheme of the present invention, a preferred method for the development of a diagnostic test comprises the following steps:
1. analyse appropriate human cells for surface peptides particularly using MALDI-MS directly on cells or, alternatively, using MALDI-MS on cell fractions especially MHC fractions, or alternatively, eluting peptides from the cell surface prior to analysis particularly by MALDI-MS or ESI-MS.
2. either, produce a profile of peptides which appear on the cell surface and, if necessary, compare with other profiles to identify an abnormality or disease associated with the human cells, or, determine sequence of specific peptides on the cell surface as a means to determining specific peptides which might used to identify an abnormality or disease associated with the human cells.

For all embodiments of the present invention it is not critical to obtain complete sequences to determine the identity of peptides due to facile access to, and increasing content within, various protein sequence databases. A small amount of internal sequence information (which can be as little as 2-3 residues) in conjunction with the known mass of the parent ion may be sufficient to characterise a peptide species. It is recognised that algorithms are available to search databases of uninterrupted fragment ion data sets. Significant examples of these include the ProteinLynx Global Server search algorithm (Micromass, Wythenshawe, UK), Mascot from Matrix Science [www.matrix.com] or the Protein Prospector suite of programmes [http://prospector.ucsf.edu] or similar. Such programmes simulate fragmentation of sequence entries in the databases with masses of the peptide of interest.

The present invention is further described by the following non-limiting examples.

### Example 1

### Method for detecting peptides bound to cell surface HLA DR using MALDI-tof.

EBV transformed human B cell lines JESTHOM and BSM were obtained from ECACC (Porton Down, UK). B cell line JESTHOM is homozygous for HLA-DR1*0101 and line BSM is homozygous for DRB1*0401. Cell lines were cultured *in vitro* using conditions recommended by the supplier. Mouse NSO cells (ECACC #85110503) cultured using the suppliers recommended conditions were used as a negative control cell line.

Synthetic peptides were obtained from Zeneca (Zeneca LSM, Northwich, UK). Peptide HA1 was a 13mer corresponding to residues 307-319 of influenza virus hemagglutinin protein (Rothbard, J.B. et al (1988) *Cell* 52: 515). Peptide MP1 was 13mer corresponding to residues 17-29 of influenza virus matrix protein [Rothbard, J.B. et al (1988) *ibid*]. Whilst both peptides are known to bind a number of different HLA-DR specificities, differential binding is seen with the DR4 allotype. MP1 binds to DR4 whereas MP1 shows no significant binding to the DR4 specificity [Busch R. et al (1990) *Int. Immunol*. 2: 443].

For some experiments analogue peptides containing a labile biotin linker moiety were used. For these the linker biotin-HPDP (Pierce, Chester, UK) was coupled to an N-terminal cysteine residue to yield peptides HA1B and MP1B. With the exception of the additional cysteine residue, the sequences are otherwise identical to HA1 and MP1. Coupling was achieved using protocols provided with the biotin-HPDP (Pierce, Chester, UK), except that purification of the coupled peptide was conducted using a HPLC and a standard elution profile. Inhibition experiments were conducted using purified monoclonal antibody LB3.1 [ATCC number HB-298]. The antibody was purified from hybridoma LB3.1 conditioned medium using protein A sepharose (Millipore, Conset, UK) affinity chromatography and procedures recommended by the supplier. The hybridoma had been maintained using standard conditions. LB3.1 was co-incubated with the peptide and cells at a maximum concentration of 10□g/ml.

Cells were paraformaldyhyde fixed before treatment with different synthetic peptides in test and control experiments. Experiments were conducted using 3x 10⁶ cells in 50□I complete medium added to 150□I peptide binding buffer (50mM Phosphate citrate buffer at pH 4.0; 0.1% NP40) containing peptide at a final concentration of 50□M. Incubation was conducted at 37°C for 24 hours. For assay, peptide treated cells were harvested by centrifugation and washed three times using PBS. The cells were placed into a 10□I aliquot of 50% acetonitrile-0.1% trifluroacetic acid in a 0.5ml microfuge tube. Each sample was subsequently mixed vigorously for 5 s before sample spotting to the MALDI instrument sample plate. The two-layer method of sample spotting was used, where 1□I acid matrix solution [0.1M sinapinic acid in a 1:1:1 mixture of acetonitrile, methanol and water] was dried onto the MS sample plate. This was followed by 1□I of the cells and a further 1□I of sinapinic acid matrix solution.

In experiments using synthetic peptides containing a labile linker mass-tag, removal of the linker was achieved by incubation of the cells in PBS containing 100mM □-mercaptoethanol (BME). Incubation with BME was conducted at 37°C for 30 minutes and cells washed 3 times using PBS before application to the MALDI sample plate as previously.

A Voyager DE mass spectrometer (Perseptive Biosystems, Foster City, CA, USA) was used in positive ion mode. The instrument was calibrated with a mixture of horse cardiac apomyglobin and bovine serum albumin (Sigma, Poole, UK) and checked less than 30 minutes before each analysis to be within 0.1% mass accuracy for each standard. The sample spots were air dried and analysed in positive ion mode. The delayed extraction was set to 150ns at 25kV. The low mass gate was set to 600Da. A total of 125 laser shots were accumulated from each sample.

Predicted mass peaks for peptides HA1 and MP1 were identified in spectra generated from JESTHOM cells. In contrast, only peptide HA1 could be identified from HLA-DR4 expressing BSM cells. In experiments using labelled peptides, mass shifts of the peptide peak was identified in the spectra of BME treated cells. Spectra from LB1.1 incubated cells (inhibition experiments) showed that the antibody treatment could not abolish peptide binding although some evidence of reduced relative ion abundance could be found in 3-way competition assays using peptide, labelled peptides and LB1.1 antibody. Spectra from mouse NSO cells treated with peptides, or peptide combinations showed very low abundance of target ion peaks suggesting non-specific interaction and or insufficient washing during cell processing.

### Example 2

### Method for analysis of MHC DR bound peptides using MALDI-tof

HLA-DR1*0101 was purified from JESTHOM cell membranes by immunoaffinity chromatography. HLA DRB1*0401 was purified from BSM cells. Solubilised cell membranes were prepared and processed as previously described [Gorga et al (1987) *J. Biol. Chem.* 262: 16087-16094; Sette et al (1989) *J. Immunol*.42: 35-40]. The anti-DR monoclonal antibody LB3.1 [ATCC number HB-298] was used as the immunoaffinity reagent. The antibody was purified from hybridoma LB3.1 conditioned medium using protein A sepharose (Millipore, Conset, UK) affinity chromatography and procedures recommended by the supplier. The hybridoma had been maintained using standard conditions. LB3.1 antibody was coupled to sepharose 4B (Pharmacia Biotech, St. Albans, UK) using the Linx system provided by InVitrogen (InVitrogen, Groningen NL) and conditions recommended by the supplier.

Synthetic peptides HA1 and MP1 as described in example 1 were incubated with a 50□I aliquot of the HLA-DR1*0101 preparation. Peptides were incubated in peptide binding buffer (50mM Phosphate citrate buffer at pH 4.0; 0.1 % NP40) at a final concentration of 50□M for 26 hours at 37°C. Unbound peptide was removed by ultrafiltration using microcon centrifugal filtration cells (Millipore, USA) and multiple cycles (minimum of four) of washing with PBS. The final volume was reduced to 20□I. In some experiments peptides were eluted from the MHC before MALDI-tof analysis. In this case elution was conducted by extraction with a solution of 0.1% TFA in H₂O. The eluate was evaporated to dryness and resuspended directly using MALDI matrix solution as per example 1. In other experiments the MHC-peptide complex was applied to the instrument sample plate resuspended in matrix solution.

Predicted mass peaks for peptides HA1 and MP1 were identified in spectra generated from HLA-DR1*0101 preparations. In contrast, only the HA1 mass peak could be identified in spectra from DRB1*0401 preparations.

### Example 3

### Method for analysis of cell surface peptides following treatment of cells with whole protein.

Human dendritic cells were enriched from 40ml peripheral blood samples obtained from healthy donors. The blood was anticoagulated using heparin and a mononuclear cell fraction prepared using histopaque 1077 (Sigma, Poole, UK) density gradient medium and conditions recommended by the supplier. Dendritic cells were obtained by negative selection using an immunomagnetic separation procedure with all reagents and conditions provided by Mitenyi Biotec (Bisely, UK). The dendritic cells were eluted into multiwell tissue culture dishes for treatment with protein antigen. Cells were maintained in RPMI medium supplemented with 10% (v/v) foetal bovine serum and standard antibiotics during the course of the experiments. All reagents were from Life Technologies (Paisley, UK).

A preparation of recombinant staphylokinase was used in these studies. The wild-type staphylokinase gene was synthesised under contract by Genosys Biotechnologies Ltd (Cambridge, UK). The gene was constructed by polymerase chain reaction using overlapping synthetic primers and the sequence as given by Collen et al [Collen D. (1996) *Circulation* 94: 197-206]. The synthetic gene was cloned as a 453 bp EcoRI-HinDIII restriction fragment into bacterial expression vector pMEX (MoBiTec, Gottingen, Germany). The pMEX/staphylokinase gene was transformed into competent E.*coli* strain TG1 by standard techniques and a single transformed clone secreting active staphylokinase was selected using a fibrin plate assay [Astrup, T. et al (1952) *Arch. Biochem. Biophys.* 40: 346-351; Collen, D. et al (1992) *Fibrinolysis* 6: 203-213]. The best expressing clone was grown up and recombinant protein was purified from the culture supernatant using sequential column chromatography and methods described previously [Collen, D. et al (1992) *ibid*; Schlott, B. et al (1994) *Biotechnology* 12: 185-189].

Dendritic cells were incubated with purified staphylokinase at a concentration of 100□g/ml and incubation conducted overnight. In some experiments, antigen processing was blocked using inhibitor cocktails added to the culture medium. Inhibitors were added 1 hour before the addition of the test protein at the following concentrations: ammonium chloride 50mM; sodium azide 1mg/ml; chloroquine and colchicine 500□M. All compounds were from Sigma (Sigma, Poole, UK).

Following protein treatment, cells were removed from culture dishes and washed using 3 cycles of centrifugation and PBS treatment to remove all medium and extraneous protein. Cells were processed for MALDI-tof analysis as given in example 1. Spectra were collected and analysed for the presence peptides attributable to the test protein. These were identified by comparison to spectra obtained from control cells not treated with staphylokinase.

Ion peaks attributed to staphylokinase were identified in spectra obtained from cells treated with staphylokinase and not seen in the spectra of cells treated with metabolic inhibitors or control cells not treated with staphylokinase.

### Example 4

### Method for determination of peptide sequences derived from treatment of cells with whole protein.

Cells were treated with the protein of interest according the method of Example 3. Peptides were eluted from the cells by multiple extraction cycles (4-6) with a solution of 5% acetonitrile, 0.1% formic acid. The presence of sequences attributable to the test protein was determined using ESI-MS/MS.

Eluted samples were dried and re-suspended in a solution of 0.1% formic acid. The entire crude eluate was separated by means of a modular CapLC system (Micromass, Wythenshawe, UK) connected directly to the Z-spray source of the mass spectrometer. Samples were loaded onto a C18 pre-column at a flow rate of 30□L per minute and desalted for 3 minutes using a solution of 0.1% formic acid. The flow rate was reduced to 1□L per minute and re-directed onto a C18 180mm pepmap column. The column was eluted using a standard elution gradient ranging from a solvent solution of 95% H₂O, 5% acetonitrile, 0.1% formic acid to a solvent solution of 5% H₂O, 95% acetonitrile, 0.1% formic acid.

Electrospray MS and MS/MS data were acquired on a Micromass Q-Tof 2 instrument (Micromass, Wythenshawe, UK) fitted with a Z-spray nanoflow electrosparay ion source. The instument was operated in the positive ion mode with a source temperature of 80°C, a counter gas flow rate of 40L/hour and with a potential of 2800V applied to the nanospray continuous LC probe. All data were acquired with the instrument operating in automatic data dependent switching mode.

The instrument was calibrated with a two point calibration selected fragment ions that resulted from the collision-induced decomposition of glufibrinopeptide b. All data was processed automatically by means of ProteinLynx software, protein identification was achieved by analysis with the ProteinLynx Global Server search algorithm (Micromass, Wythenshawe, UK).

Manual data acquisition was performed by injecting the crude sample into the instrument via borosilicate needles. The samples were adjusted to a concentration of approximately 1□M. Samples were desalted prior to injection by use of C18 ZipTip disposable minicolumns (Millipore, USA).

Spectra were collected and analysed for the presence peptide masses attributable to the test protein. These were identified by comparison to spectra obtained from control cells including cells in which antigen processing was blocked. Following treatment with staphylokinase peptide masses attributed staphylokinase were identified. Equivalent ion peaks were not evident in cells not treated with staphylokinase or with cells treated with metabolic inhibitors.

## Claims

1. A method for detecting immunogenic peptides in association with MHC molecules on the surface of cells, or exosomal vesicles thereof, which derive from a protein or polypeptide having one or more T-cell epitopes within its amino acid sequence, the method comprising the subsequent steps:
(i) contacting cells from a combination of human cell lines or cell samples, providing a mixture of MHC class II restricted epitopes, with said protein or polypeptide, thus generating a repertoire of surface peptides on that cells or exosomal vehicles thereof, which is different from the repertoire of surface peptides displayed on reference cells which have not been contacted, wherein said peptides on the surface bind to multiple different MHC molecules.
(ii) cluting said peptides from the surface of said cells or from MHC molecules,
(iii) analyzing said cells or exosomal vesicles therein by detecting said peptides on the cell surface by using MALDI-MS or ESI-MS, and
(iv) assigning said peptides to the sequence of said protein or polypeptide according to standard methods.

2. A method of claim 1, wherein said peptides on the surface of cells are products of intracellular peptidase and trafficking pathway.

3. A method of claim 1 or 2, wherein said MHC molecules have been enriched prior to peptide analysis.

4. A method of any of the claims 1- 3, wherein the cells are selected from the group consisting of human dendritic cells, human antigen presenting cells and cells from human cell lines engineered to produce MHC molecules.

5. A method of any of the claims 1 to 4, wherein said protein or polypeptide is an exogenous protein or polypeptide.

6. A method of any of the claims 1 to 4, wherein said protein or polypeptide is an endogenous protein or polypeptide.

7. A method of designing new sequence variants of a pharmaceutical protein or polypeptide having a reduced immunogenicity than the non-modified protein or polypeptide, the method comprising the subsequent steps:
(i) detecting immunogenic peptides, which derive from said protein or polypeptide and bind to MHC molecules, by the method of any of the claims 1 - 6, and
(ii) eliminating or reducing the binding of said immunogenic peptides to said MHC molecules by substituting, deleting or inserting one or more amino acid residues within said peptide within said pharmaceutical protein or polypeptide.

8. A method of designing new sequence variants of a pharmaceutical protein or polypeptide having an enhanced immunogenicity than the non-modified protein or polypeptide, the method comprising the subsequent steps:
(i) detecting immunogenic peptides, which derive from said protein or polypeptide and bind to MHC molecules, by the method of any of the claims 1 - 6, and
(ii) enhancing the binding of said immunogenic peptides to said MHC molecules by substituting, deleting or inserting one or more amino acid residues within said peptide within said pharmaceutical protein or polypeptide.

9. A method of claim 7 or 8 additionally comprising the step:
(iii) testing the modified peptides for binding to said cell surface as indicated in claim 1.

10. A method of claims 7-9, wherein the pharmaceutical protein or polypeptide is a vaccine.

## Patentansprüche

1. Verfahren zum Nachweis immunogener Peptide in Verbindung mit MHC-Molekülen auf der Oberfläche von Zellen oder deren exosomalen Vesikeln, die von einem Protein oder Polypeptid mit einem oder mehreren T-Zell-Epitopen innerhalb seiner Aminosäuresequenz stammen, wobei das Verfahren die nachstehenden Schritte umfasst:
(i) In-Kontakt-bringen von Zellen aus einer Kombination von menschlichen Zelllinien oder Zellproben, die ein Gemisch von MHC-Klasse-IIrestringierten Epitopen bereitstellen, mit dem genannten Protein oder Polypeptid, so dass ein Repertoire von Oberflächenpeptiden auf den Zellen oder deren exosomalen Vesikeln erzeugt wird, das sich von dem Repertoire an Oberflächenpeptiden unterscheidet, die auf Bezugszellen dargeboten werden, die nicht in Kontakt gebracht wurden, wobei die genannten Peptide auf der Oberfläche an mehrere verschiedene MHC-Moleküle binden,
(ii) Eluieren der genannten Peptide von der Oberfläche der Zellen oder von MHC-Molekülen,
(iii) Analysieren der genannten Zellen oder der exosomalen Vesikel darin durch Nachweisen der Peptide auf der Zelloberfläche unter Verwendung von MALDI-MS oder ESI-MS und
(iv) Zuordnen der genannten Peptide zu der Sequenz des genannten Proteins oder Polypeptids gemäß Standardverfahren.

2. Verfahren nach Anspruch 1, wobei die genannten Peptide auf der Oberfläche von Zellen Produkte des intrazellulären Peptidase- und Transport- (Trafficking-) Wegs sind.

3. Verfahren nach Anspruch 1 oder 2, wobei die genannten MHC-Moleküle vor der Peptidanalyse angereichert werden.

4. Verfahren nach einem der Ansprüche 1 bis 3, wobei die Zellen aus der Gruppe ausgewählt sind, die aus menschlichen dendritischen Zellen, menschlichen antigenpräsentierenden Zellen und Zellen aus menschlichen Zelllinien ausgewählt sind, die genetisch verändert wurden, so dass sie MHC-Moleküle produzieren.

5. Verfahren nach einem der Ansprüche 1 bis 4, wobei das genannte Protein oder Polypeptid ein exogenes Protein oder Polypeptid ist.

6. Verfahren nach einem der Ansprüche 1 bis 4, wobei das genannte Protein oder Polypeptid ein endogenes Protein oder Polypeptid ist.

7. Verfahren zum Design neuer Sequenzvarianten eines pharmazeutischen Proteins oder Polypeptids mit geringerer Immunogenität als das unmodifizierte Protein oder Polypeptid, wobei das Verfahren die nachstehenden Schritte umfasst:
(i) Nachweisen immunogener Peptide, die von dem genannten Protein oder Polypeptid stammen und an MHC-Moleküle binden, durch das Verfahren nach einem der Ansprüche 1 bis 6 und
(ii) Beseitigen oder Verringern der Bindung der genannten immunogenen Peptide an die genannten MHC-Moleküle durch Ersetzen, Deletieren oder Hinzufügen von einem oder mehreren Aminosäureresten innerhalb des Peptids innerhalb des genannten pharmazeutischen Protein oder Polypeptids.

8. Verfahren zum Design neuer Sequenzvarianten eines pharmazeutischen Proteins oder Polypeptids mit stärkerer Immunogenität als das unmodifizierte Protein oder Polypeptid, wobei das Verfahren die nachstehenden Schritte umfasst:
(i) Nachweisen immunogener Peptide, die von dem genannten Protein oder Polypeptid stammen und an MHC-Moleküle binden, durch das Verfahren nach einem der Ansprüche 1 bis 6 und
(ii) Verstärken der Bindung der genannten immunogenen Peptide an die genannten MHC-Moleküle durch Ersetzen, Deletieren oder Hinzufügen von einem oder mehreren Aminosäureresten innerhalb des Peptids innerhalb des genannten pharmazeutischen Protein oder Polypeptids.

9. Verfahren nach Anspruch 7 oder 8, das zusätzlich den Schritt umfasst:
(iii) Testen der modifizierten Peptide hinsichtlich ihrer Bindung an die genannte Zelloberfläche, wie in Anspruch 1 angegeben.

10. Verfahren nach den Ansprüchen 7 bis 9, wobei das pharmazeutische Protein oder Polypeptid ein Vakzin ist.

## Revendications

1. Procédé pour détecter des peptides immunogènes en association avec des molécules MHC sur la surface de cellules ou sur leurs vacuoles exosomales, qui sont dérivés à partir d'une protéine ou d'un polypeptide comportant un ou plusieurs épitopes de cellule T à l'intérieur de sa séquence acide aminé, le procédé comprenant les étapes qui suivent :
(i) mise en contact des cellules en provenance d'une combinaison d'une ligne de cellules humaines ou d'échantillons de cellules, constitution d'un mélange d'épitopes restreints MHC de classe II avec ladite protéine ou ledit polypeptide, d'où ainsi la génération d'un répertoire de peptides de surface sur ces cellules ou sur leurs vacuoles exosomales, qui est différent du répertoire de peptides de surface affiché sur des cellules de référence qui n'ont pas été mises en contact, où lesdits peptides sur la surface se lient à de multiples molécules MHC différentes ;
(ii) élution desdits peptides de la surface desdites cellules ou de molécules MHC ;
(iii) analyse desdites cellules ou desdites vacuoles exosomales dedans en détectant lesdits peptides sur la surface de cellules en utilisant MALDI-MS ou ESI-MS ; et
(iv) assignation desdits peptides à la séquence de ladite protéine ou dudit polypeptide conformément à des procédés standards.

2. Procédé selon la revendication 1, dans lequel lesdits peptides sur la surface de cellules sont des produits de peptidase intracellulaire et de passage de cheminement.

3. Procédé selon la revendication 1 ou 2, dans lequel lesdites molécules MHC ont été enrichies avant une analyse de peptide.

4. Procédé selon l'une quelconque des revendications 1 à 3, dans lequel les cellules sont sélectionnées à partir du groupe qui comprend des cellules dendritiques humaines, des cellules présentant un antigène humain et des cellules en provenance de lignes de cellules humaines soumises à ingénierie afin de produire des molécules MHC.

5. Procédé selon l'une quelconque des revendications 1 à 4, dans lequel ladite protéine ou ledit polypeptide est une protéine ou un polypeptide exogène.

6. Procédé selon l'une quelconque des revendications 1 à 4, dans lequel ladite protéine ou ledit polypeptide est une protéine ou un polypeptide endogène.

7. Procédé de conception de nouvelles variantes de séquence d'une protéine ou d'un polypeptide pharmaceutique présentant une immunogénicité réduite vis-à-vis de la protéine ou du polypeptide non modifié, le procédé comprenant les étapes qui suivent :
(i) détection de peptides immunogènes qui sont dérivés à partir de ladite protéine ou dudit polypeptide et qui se lient sur des molécules MHC, au moyen du procédé selon l'une quelconque des revendications 1 à 6 ; et
(ii) élimination ou réduction de la liaison desdits peptides immunogènes sur lesdites molécules MHC en substituant, supprimant ou insérant un ou plusieurs résidus d'acide aminé à l'intérieur dudit peptide à l'intérieur de ladite protéine ou dudit polypeptide pharmaceutique.

8. Procédé de conception de nouvelles variantes de séquence d'une protéine ou d'un polypeptide pharmaceutique présentant une immunogénicité rehaussée vis-à-vis de la protéine ou du polypeptide non modifié, le procédé comprenant les étapes qui suivent :
(i) détection de peptides immunogènes qui sont dérivés à partir de ladites protéine ou dudit polypeptide et qui se lient sur des molécules MHC, au moyen du procédé selon l'une quelconque des revendications 1 à 6 ; et
(ii) rehaussement de la liaison desdits peptides immunogènes sur lesdites molécules MHC en substituant, supprimant ou insérant un ou plusieurs résidus d'acide aminé à l'intérieur dudit peptide à l'intérieur de ladite protéine ou dudit polypeptide pharmaceutique.

9. Procédé selon la revendication 7 ou 8, comprenant de façon additionnelle l'étape de :
(iii) test des peptides modifiés pour une liaison sur ladite surface de cellules comme indiqué selon la revendication 1.

10. Procédé selon les revendications 7 à 9, dans lequel la protéine ou le polypeptide pharmaceutique est un vaccin.
